# EUROPÄISCHE PATENTSCHRIFT

(11) **EP 0 866 792 B1**
(45) Veröffentlichungstag und Bekanntmachung des Hinweises auf die Patenterteilung: **17.04.2002**
(21) Anmeldenummer: 96943936.3
(22) Anmeldetag: 12.12.1996
(51) Int. Cl.: C07D 405/12

(54) **VERFAHREN ZUR HERSTELLUNG EINES KRISTALLINEN POLYMORPHS VON TERAZOSIN-HYDROCHLORID**
PROCESS FOR PREPARING A CRYSTALLINE POLYMORPH OF TERAZOSIN HYDROCHLORIDE
PROCEDE POUR PREPARER UN POLYMORPHE CRISTALLIN DE CHLORHYDRATE DE TERAZOSINE

(30) Priorität: 13.12.1995 DE 19546573
(43) Veröffentlichungstag der Anmeldung: 30.09.1998
(73) Patentinhaber: CU Chemie Uetikon GmbH, 77933 Lahr (DE)
(72) Erfinder: BAUER, Christophe, F-67000 Strasbourg (FR); GRUNENWALD, Jean-Marie, F-67230 Kertzfeld (FR)
(74) Vertreter: Glawe, Delfs, Moll & Partner
(86) Internationale Anmeldenummer: EP9605570
(87) Internationale Veröffentlichungsnummer: WO9721705

(56) Entgegenhaltungen:
- EP-A- 0 683 167
- US-A- 5 294 615
- CHEMICAL ABSTRACTS, vol. 119, no. 28, 1993 Columbus, Ohio, US; abstract no. 95556y, Seite 1103; Spalte 2; XP002025696 & JP 00 578 352 A (SUMIFA FUAIN KEMU KK) 30.März 1993

## Beschreibung

Die Erfindung betrifft ein Verfahren zur Herstellung eines kristallinen Polymorphs von 1-(4-Amino-6,7-dimethoxy-2-chinazolinyl)-4-(2-Tetrahydrofuroyl)-piperazin-Monohydrochlorid, abgekürzt als Terazosin-Hydrochlorid bezeichnet.

Die Verbindung 1- (4-Amino-6,7-dimethoxy-2-chinazolinyl) -4-(2-Tetrahydrofuroyl)-piperazin ist unter dem allgemeinen Namen Terazosin bekannt. Für das Monohydrochlorid dieser Verbindung sind mehrere verschiedene wasserfreie Kristallformen (Polymorphe), eine kristalline Dihydratform sowie ein kristallines Addukt mit Methanol bekannt. Diese Verbindung und ihre verschiedenen Polymorphe zeichnen sich durch pharmazeutische Anwendungsmöglichkeiten, z.B. als Blutdruck senkender Wirkstoff, aus.

Die japanische Patentanmeldungsschrift JP-A-05-78352 beschreibt mehrere Kristallformen von Terazosin-Hydrochlorid und deren Herstellungswege. Ein dort als A-2 bezeichnetes kristallines Polymorph ist durch ein Pulverröntgenbeugungsspektrum mit charakteristischen Peaks bei 7°, 12°, 20,3° und einem Dublett bei 24,0° gekennzeichnet und dürfte mit dem Produkt des erfindungsgemäßen Verfahrens identisch sein. Es wird gemäß dieser Schrift wie folgt hergestellt: Zunächst wird die freie Terazosinbase hergestellt. Diese wird in das kristalline Methanoladdukt ihres Hydrochlorids überführt. Anschließend wird durch Trocknung das Kristallmethanol entfernt, wobei Temperaturen um 110°C notwendig sind. Die dadurch erhaltene, weitgehend methanolfreie, sehr hygroskopische Verbindung wird durch Behandlung mit Ethanol oder Gemischen aus Ethanol und Methylenchlorid in das erwünschte, wenig hygroskopische kristalline Polymorph überführt.

Das kristalline Polymorph, auf das sich die Erfindung bezieht, ist auch in der nicht vorveröffentlichten EP-0 708 104 A1 beschrieben und wird dort als "Form IV" bezeichnet. Bei dem in dieser Schrift vorgeschlagenen Herstellungsverfahren wird N-(2-Tetrahydrofuroyl)-piperazin, (dessen Herstellung durch Hydrieren von N-(2-Furoyl)-piperazin bereits aus US-A-4 026 894 bekannt ist) mit 4-Amino-2-chlor-6,7-dimethoxy-chinazolin gekuppelt, und zwar durch eine Behandlung, die ein mehrstündiges Kochen in einem hochsiedendem Lösungsmittel umfaßt, wodurch man das Terazosin-Hydrochlorid in der Kristallform IV erhält. Dieses Produkt wird anschließend in das Dihydrat umgewandelt und getrocknet. Ein Nachteil dieses Verfahrens besteht darin, daß es ein mehrstündiges Kochen in einem hochsiedenden Lösungsmittel (z.B. Methoxyethanol, das gesundheitlich nicht unbedenklich ist) umfaßt, wodurch zwangsläufig Verunreinigungen entstehen und eine unerwünschte Dunkelfärbung eintritt. Die Kristallform IV fällt somit nicht in der für pharmakologische Anwendung erwünschten optimalen Reinheit an, sondern bedarf einer anschließenden Weiterbehandlung (Dihydratbildung), die eine Reinigung bewirkt, wobei zusätzlich eine Behandlung mit Kieselgur erfolgt.

Andere kristalline Polymorphe von Terazosin-Hydrochlorid und ihre Herstellungsverfahren sind in US-A-5,294,615 und US-A-5,362,730 (entsprechend EP-A-0 623 612) sowie in US-A-5,412,095 beschrieben und werden dort als Form II bzw. Form III bezeichnet und jeweils durch die charakteristischen Peaks ihrer Pulverröntgenbeugungsspektren gekennzeichnet.

Der Erfindung liegt die Aufgabe zugrunde, ein Verfahren anzugeben, mit dem das bereits unter der Bezeichnungen A-2 bzw. Form IV bekannte kristalline Polymorph von Terazosin-Hydrochlorid auf besonders einfache Weise und in besonders reiner Form erhalten werden kann.

Die Aufgabe wird durch das im Anspruch 1 angegebene Herstellungsverfahren gelöst.

Das erfindungsgemäße Verfahren geht aus von dem stabilen kristallinen Addukt aus Terazosin-Hydrochlorid und Methanol, welches mit organischen polaren Lösungsmitteln oder Lösungsmittelgemischen aus der Familie der Alkohole und der Ketone, ohne vorherige Zwischentrocknung, behandelt wird. Dabei hat sich gezeigt, daß auch methanolfeuchtes Methanoladdukt, so wie es bei seiner Herstellung anfällt, ohne Nachteil einsetzbar ist, und daß dabei der Restmethanolgehalt des Endproduktes sich in einem für pharmakologische Anwendungen noch vertretbaren Rahmen hält.

Ein wesentlicher Vorteil des erfindungsgemäßen Verfahrens gegenüber dem oben genannten, aus JP-A-05-78352 bekannten Verfahren besteht somit darin, daß die Zwischentrockung bei Temperaturen um 110°C entfällt. Dieser Zwischentrocknungsschritt des bekannten Verfahrens hat zwei wesentliche Nachteile. Zum einen entsteht durch die Trocknung eine sehr hygroskopische Verbindung. Zum anderen kann die mehrstündige thermische Belastung bei den Trocknungstemperaturen zu beginnender Zersetzung des Produktes und damit zur Entstehung von Verunreinigungen führen, was für die Verwendung des Produktes als Pharmawirkstoff nachteilig ist. Diese Nachteile werden durch das erfindungsgemäße Verfahren vermieden.

Das nach dem erfindungsgemäßen Verfahren hergestellte kristalline Polymorph hat gegenüber anderen wasserfreien Polymorphen von Terazosin-Hydrochlorid wesentliche Vorteile. Es zeichnet sich durch eine geringere Hygroskopizität sowie durch eine geringere Retentionsfähigkeit für Restlösungsmittel aus, was für die Verwendung als pharmakologischer Wirkstoff von großem Vorteil ist.

Ein Ausführungsbeispiel des erfindungsgemäßen Herstellungsverfahrens wird nachfolgend beschrieben.

### 1. Herstellung des kristallinen Addukts aus Terazosin-Hydrochlorid und Methanol.

37,6g 4-Amino-2-(1-piperazinyl)-6,7-dimethoxychinazolin und 15,8g Triethylamin werden in 400ml Methylenchlorid vorgelegt. Die Suspension wird auf -5°C abgekühlt. Unter Rühren werden 21,0g Tetrahydrofuran-2-carbonsäurechlorid zugetropft. Die Suspension wird zwei Stunden bei 0°C nachgerührt, danach wird das Lösungsmittel unter Vakuum abdestilliert. Zum Rückstand werden 200ml Methanol und 200ml Aceton gegeben; die entstandene Suspension wird 1/2 Stunde unter Rühren auf Rückfluß gehalten; danach wird sie auf 0°C abgekühlt. Das Produkt wird durch Filtration isoliert; man erhält 78g feuchte Terazosinbase.

Diese wird in 400ml Methanol und 400ml Methylenchlorid aufsuspendiert; unter Rühren werden 23,8g einer 20%igen Lösung von Chlorwasserstoff in Methanol zugetropft. Dabei entsteht eine Lösung, die klarfiltriert wird. Unter Vakuum wird das Methylenchlorid selektiv abdestilliert, dabei fällt das Produkt aus. Die Suspension wird 3 Stunden bei Raumtemperatur nachgerührt, danach wird das Produkt durch Filtration isoliert. Man erhält 74g feuchtes Terazosin-Hydrochlorid Methanoladdukt, das wahlweise bei 50°C unter Vakuum getrocknet werden kann; dabei erhält man 49,0g weißes Kristallpulver, das ca. 1 Mol Methanol pro Mol Terazosin-Hydrochlorid enthält. Eine zusätzliche Reinigung des Methanoladduktes ist durch Umkristallisation aus Methanol möglich.

Die insoweit beschriebenen Verfahrensstufen entsprechen im Prinzip dem aus der bereits genannten JP-A-05-78352 bekannten Herstellungsverfahren für das dort als "Typ M" bezeichnete Methanoladdukt.

### 2. Herstellung des kristallinen Polymorphs.

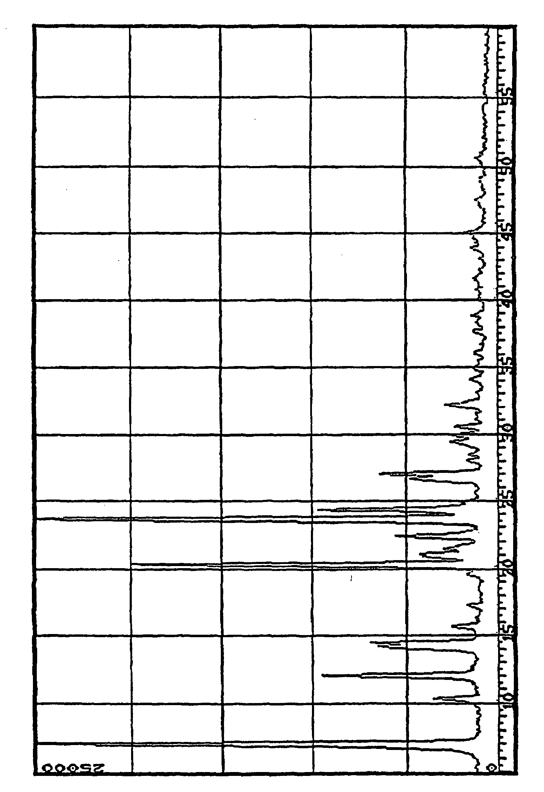

10g des kristallinen Addukts, dessen Herstellung vorstehend beschrieben wurde, werden in 100ml Ethanol, das mit 2-Butanon vergällt ist, suspendiert. Die Suspension wird unter Rühren erhitzt und 2 Stunden lang am Rückfluß des Ethanols gehalten, wobei die Rückflußtemperatur beispielsweise 78°C - 80°C betragen kann. Anschließend wird die Suspension auf 20°C abgekühlt. Das Produkt wird durch Filtration isoliert und bei 50°C unter Vakuum getrocknet. Man erhält 8,4g Kristallpulver, dessen Pulverröntgendiffraktionsspektrum in der Abbildung wiedergegeben ist. Eine Bestimmung des Restgehaltes an Lösungsmittel ergibt, daß das Kristallpulver typischerweise weniger als 100ppm Methanol und weniger als 100ppm Ethanol enthält.

Das mit dem polaren Lösungsmittel zu behandelnde Methanolat des Terazosin-Monohydrochlorids ist in dem polaren Lösungsmittel nur geringfügig löslich. Die Menge des verwendeten polaren Lösungsmittels wird so bemessen, daß möglichst wenig des Methanolats in Lösung geht, so daß die weitaus überwiegende Menge des zu behandelnden Produktes als Suspension in dem polaren Lösungsmittel vorliegt. Dies hat wesentliche Vorteile für die Verfahrensdurchführung, insbesondere für die Wiedergewinnung des festen Produktes durch einfache mechanische Trennung, wie z.B. Filtrieren. Die Behandlung in dem polaren Lösungsmittel wird vorzugsweise bei einer erhöhten Temperatur durchgeführt.

Die Kristallform des mit dem Verfahren erhaltenen Produktes ist durch das in der Abbildung dargestellte Pulverröntgendiffraktionsspektrum gekennzeichnet. Dieses enthält charakteristische Peaks bei den folgenden, in Winkelgrad angegebenen Werten von 2 θ: 7,04; 10,30; 12,02; 14,12; 14,44; 20,34; 22,34; 22,62; 23,58; 24,28 und 26,96, (alle Werte ± 0,2).

Die Erfindung ist nicht auf die Einzelheiten des beschriebenen Ausführungsbeispiels beschränkt. Insbesondere kann als polares Lösungsmittel, mit dem das kristalline Methanolat des Terazosin-Hydrochlorid behandelt wird, jedes Lösungsmittel aus der Gruppe der Alkohole mit zwei bis sechs Kohlenstoffatomen oder der Ketone mit drei bis sechs Kohlenstoffatomen, oder eine Mischung aus zwei oder mehr dieser Lösungsmittel, verwendet werden.

## Patentansprüche

1. Verfahren zur Herstellung eines kristallinen wasserfreien Polymorphs von 1-(4-Amino-6,7-dimethoxy-2-chinazolinyl)-4-(2-tetrahydrofuroyl)-piperazin-Monohydrochlorid (Terazosin-Monohydrochlorid) der Kristallform F4, deren Pulverröntgendiffraktionsspektrum charakteristische Peaks bei folgenden Werten von 2 θ (in Winkelgrad) aufweist: 7,04; 10,30; 12,02; 14,12; 14,44; 20,34; 22,34; 22,62; 23,58; 24,28; 26,96; jeder Wert ± 0,2,
**gekennzeichnet durch** folgende Schritte:
- Herstellung des kristallinen Adduktes aus Terazosin-Monohydrochlorid und Methanol;
- Behandlung des kristallinen Adduktes in Suspension mit einem polaren Lösungsmittel, ausgewählt aus der Gruppe der Alkohole mit zwei bis sechs Kohlenstoffatomen, der Ketone mit drei bis sechs Kohlenstoffatomen, oder einer Mischung daraus,
- Isolierung des festen Produktes aus der Suspension,
wobei die Behandlung in Suspension bei einer erhöhten Temperatur erfolgt, die ausreichend hoch gewählt ist, damit sich die Kristallform F4 bildet.

2. Verfahren nach Anspruch 1, **dadurch gekennzeichnet, daß** das Lösungsmittel Ethanol ist und die erhöhte Temperatur mindestens 78°C beträgt.

3. Verfahren nach Anspruch 1 oder 2, **dadurch gekennzeichnet, daß** die Behandlungszeit mindestens 1 Stunde, vorzugsweise ca. 2 Stunden beträgt.

## Claims

1. A method of preparing a crystalline anhydrous polymorph of 1-(4-amino-6,7-dimethoxy-2-quinazolinyl-4-(2-tetrahydrofuroyl)-piperazine-monohydrochloride (terazosin-monohydrochloride) of the crystalline form IV, the powder X-ray diffraction spectrum of which has characteristic peaks at the following values of 2 θ (in angular degree): 7.04; 10.30; 12.02; 14.12; 14.44; 20.34; 22.34; 22.62; 23.58; 24.28; 26.96; each value + 0.2, **characterised by** the following steps:
- preparing the crystalline adduct from terazosin-monohydrochloride and methanol;
- treating the crystalline adduct in suspension with a polar solvent, selected from the group of alcohols having two to six carbon atoms, the ketones having three to six carbon atoms, or a mixture thereof,
- isolating the solid product from the suspension,
wherein the treatment in suspension takes place at a raised temperature which is chosen to be sufficiently high that the crystalline form IV is formed.

2. A method according to Claim 1, **characterised in that** the solvent is ethanol and the high temperature is at least 78°C.

3. A method according to Claim 1 or 2, **characterised in that** the treatment time is at least 1 hour, preferably approximately 2 hours.

## Revendications

1. Procédé de préparation d'un polymorphe cristallin anhydre du monochlorhydrate de 1-(4-amino 6,7-diméthoxy 2-quinazolinyl) 4-(2-tétrahydrofuroyl)-pipérazine (monochlorhydrate de térazosine) de forme cristalline F4, dont le spectre de diffraction des rayons X en poudre présente des pics caractéristiques aux valeurs de 2 θ suivantes (en degrés d'angle) : 7,04 ; 10,30 ; 12,02 ; 14,12 ; 14,44 ; 20,34 ; 22,34 ; 22,62 ; 23,58 ; 24,28 ; 26,96 ; ± 0,2 pour chaque valeur,
**caractérisé par** les étapes suivantes :
- préparation du produit d'addition cristallin à partir de monochlorhydrate de térazosine et de méthanol ;
- traitement du produit d'addition cristallin en suspension dans un solvant polaire choisi dans le groupe comprenant des alcools ayant de deux à six atomes de carbone, des cétones ayant de trois à six atomes de carbone, ou bien un mélange de ces substances ;
- isolation du produit solide à partir de la suspension ;
le traitement étant effectué en suspension à une température élevée, choisie à une valeur suffisamment grande pour que la forme cristalline F4 se constitue.

2. Procédé selon la revendication 1, **caractérisé en ce que** le solvant est l'éthanol et **en ce que** la température élevée est d'au moins 78°C.

3. Procédé selon la revendication 1 ou la revendication 2, **caractérisé en ce que** la durée du traitement est d'au moins 1 heure, de préférence environ 2 heures.
